(19) Europäisches Patentamt

European Patent Office

Office européen des brevets

(11) **EP 2 861 131 B1**

(12) **EUROPEAN PATENT SPECIFICATION**

(45) Date of publication and mention
of the grant of the patent:
**21.02.2018 Bulletin 2018/08**

(21) Application number: **13742283.8**

(22) Date of filing: **10.06.2013**

(51) Int Cl.:
***A61B 5/00*** *(2006.01)*    ***A61B 5/024*** *(2006.01)*

(86) International application number:
**PCT/IB2013/054745**

(87) International publication number:
**WO 2013/190423 (27.12.2013 Gazette 2013/52)**

(54) **PHOTOPLETHYSMOGRAPHIC DEVICE AND METHOD.**

PHOTOPLETHYSMOGRAFIEVORRICHTUNG UND -VERFAHREN

DISPOSITIF PHOTOPLÉTHYSMOGRAPHIQUE ET PROCÉDÉ ASSOCIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2012 US 201261660854 P**

(43) Date of publication of application:
**22.04.2015 Bulletin 2015/17**

(73) Proprietor: **Koninklijke Philips N.V.
5656 AE Eindhoven (NL)**

(72) Inventors:
• **SCHIPPER, Alphonsus Tarcisius Jozef Maria
NL-5656 AE Eindhoven (NL)**

• **LULOFS, Klaas Jacob
NL-5656 AE Eindhoven (NL)**
• **VAN ENGEN, Pieter Geert
NL-5656 AE Eindhoven (NL)**
• **GEENEN, Koen
NL-5656 AE Eindhoven (NL)**

(74) Representative: **Roche, Denis
Société Civile SPID
Philips IP&S
33, rue de Verdun
BP 313
92156 Suresnes Cedex (FR)**

(56) References cited:
**WO-A2-2005/009221      US-A- 5 800 348
US-A- 5 846 190      US-A- 5 954 644**

Printed by Jouve, 75001 PARIS (FR)

**Description**

FIELD OF THE INVENTION

The present invention relates to a monitoring device and a monitoring method, in particular for heart rate measurements.

BACKGROUND OF THE INVENTION

Heart beat detectors or pulse oximeters, as an important species of monitoring devices, based on photoplethysmography (PPG) are well known. Light from one or more light sources (e.g. LEDs) is emitted into the tissue of a living being (e.g. a patient) and is captured by a light sensor (e.g. a photo diode).

US 5,846,190 discloses a pulse oximeter having several processing channels for measurement signals which are measured during the switched-on phase of transmission diodes and which are used to determine the oxygen saturation (SpO2) of a patient. An additional measurement channel processes an ambient light signal which was measured while the transmission diodes were switched off or their intensity modified, in the same way and thus provides a measure of the spectral composition of the ambient light interference. A useful-to-noise-signal ratio is derived from the ambient light signal and a measurement signal, which represents a measure of the signal quality and which can be compared with threshold values, where an alarm is triggered if the value falls below the threshold values.

A problem with heart beat detectors is that ambient light leaks into the sensor and disturbs the measurement. This ambient light may originate from the sun or from electrical lamps (which may emit light at the net frequency). Another problem is that noise, especially low frequent noise (e.g. 1/f noise), but also high frequent noise, disturbs the measurement as well. Furthermore, electromagnetic interference may disturb the measurement as well.

A way around this is to use synchronous detection. In synchronous detection, the to-be-measured signal (baseband signal) is modulated onto a carrier wave. A detector demodulates the signal such that the baseband signal is obtained again. The advantage of synchronous detection is that only noise and disturbance signals in a small frequency band (around the modulation frequency) may disturb the detected signal. All noise and disturbance outside the small frequency band are filtered out and therefore do not disturb the detected signal. Pulse oximeters that use synchronous detection are known, for example from US 6,912,413.

In a straightforward implementation, as e.g. known from radio receivers, synchronous detection is done by modulating the light source with a sine wave. This is good for signal quality as only noise and disturbances in a small band around the modulation frequency may disturb the detected signal. The small band must be located sufficiently high in frequency to get rid of the lower frequent disturbances (e.g. 120 Hz light). This requires the modulation frequency to at least be above this. This form of synchronous detection requires complex circuitry. Instead of a sine wave, also a square wave is being used. By filtering out the high frequent components in the detector, only the frequency band around the base frequency of the square wave is utilized.

SUMMARY OF THE INVENTION

It is an object of the present invention to provide a monitoring device and a monitoring method that do not require modulation by a sine or square wave but allow for a low frequent, low duty cycle pulsed light modulation.

In a first aspect of the present invention a monitoring device is presented comprising:

- a light source for emitting light pulses into tissue of a living being,
- a light sensor for receiving light from said tissue and generating a sensor signal,
- a filter unit for filtering said sensor signal, said filter unit comprising a switched in-phase low-pass filter and a switched out-of-phase low-pass filter,
- a control unit for controlling said light source and said filter unit such that the in-phase filter is only switched on during a second time period while the light source is switched on and that the out-of-phase filter is switched on during a first and third time period while the light source is switched off, wherein said control unit is configured to control said light source and said filter unit such that said first time period is arranged shortly before the light source gets switched on and said third time period is arranged shortly after the light source gets switched off, wherein the out-of-phase filter is configured to generate an ambient signal by integrating the sensor output during the first and third time period, and wherein the in-phase filter is configured to generate a main signal by integrating the sensor output during the second time period, and
- a subtraction unit for generating a corrected signal by subtracting the ambient signal from the main signal.

In a further aspect of the present invention a corresponding monitoring method is presented.

Preferred embodiments of the invention are defined in the dependent claims. It shall be understood that the claimed method has similar and/or identical preferred embodiments as the claimed device and as defined in the dependent claims.

The present invention is based on the idea to apply a synchronous detection scheme that does not require modulation by a sine or square wave but allows for a low frequent, low duty cycle pulsed light modulation, while still only allowing noise and disturbances in a narrow, higher frequency band to disturb the detected signal.

According to the present invention an ambient signal (i.e. generally including ambient light and noise) is measured close around the main signal. While measuring (sampling) the ambient signal, the light source is off. While measuring (sampling) the main signal the light source is on. The corrected signal is created by subtracting the ambient signal from the main signal. In this way, the undesired ambient signal is removed from the main signal. By measuring (sampling) close around the main signal, a locally increased sampling frequency is realised such that ambient signals up to a high frequency can be detected and compensated for, independent of the sampling frequency with which the main signal is sampled.

Switched low pass filters are used for the detection of both the ambient and main signal. The ambient signal thus corresponds to the out-of-phase filter signal, and the main signal corresponds to the in-phase filter signal. The output signal of the device is obtained by subtraction of the two different signals at the output of the two filters. The switched low pass filters are used to prevent aliasing and to filter out high frequent noise.

It shall be noted that the invention is generally not limited to the use of three time periods, but that an interleaved sequence of arbitrary length is possible, such as out-of-phase time period; in-phase time period; out-of-phase time period; in-phase time period; ...; out-of-phase time period. In such an embodiment the light source is only on during the in-phase time periods. All in-phase time periods are integrated in the in-phase integrator (filter), while all out-of-phase time periods are integrated in the out-of-phase integrator (filter). Preferably, the sum of the in-phase time periods is nominally equal to the sum of the out-of-phase time periods.

In an embodiment said control unit is configured to control said filter unit such that the second time period has the same length as the sum of the first and third time periods.

The sum of the length of the first and third time periods does not have to be equal to the length of the second time period. The difference may in this case be compensated for by proper weighing factors. Hence, in another embodiment said control unit is configured to control said filter unit such that the contribution of the disturbance signal during the second time period is the same as the contribution of the disturbance signal during the first and third time periods. This is where calibration comes in. To compensate for RC difference between the two filters of the filter unit, the time may deliberately be made slightly unequal in a kind of calibration procedure. The calibration aims at slightly tuning the time periods such that the contribution of the disturbance signal is the same in both filters.

In another embodiment said control unit is configured to control said filter unit such that the first time period has the same length as the third time period. Generally, it is, however, also possible that the first time period has a different length than the third time period.

In another embodiment said control unit is configured to control said light source and said filter unit such that said first time period is arranged shortly before the light source gets switched on and said third time period is arranged shortly after the light source gets switched off. This provides that the ambient light is measured during all three time periods in most similar way and contribution.

In another embodiment said control unit is configured to control said light source and said filter unit such that the time distance between the first time period and the third time period is short compared to the time period of the fastest disturbance signal to be suppressed, i.e. $t_{3rd\ period} - t_{1st\ period} << 1/f_{disturbance\_max}$.

The exact moment in time when the light source gets switched on and off is selected such that an integration of ambient light signals is made such that the integrator output (out-of-phase signal) approximates the true value of the ambient light signal at the time the light source is on. The best approximation is made if the first and third time periods are symmetrical and close to the second time period.

In another embodiment said control unit is configured to control said light source and said filter unit such that the second time period is shorter than the time duration of a light pulse.

In another embodiment the monitoring device further comprises an analog-to-digital converter coupled between said filter unit and said subtraction unit for analog-to-digital conversion of said in-phase filter signal and said out-of-phase filter signal before subtracting them or coupled to the output of said subtraction unit for analog-to-digital conversion of the output signal of said subtraction unit. Thus, the full A/D resolution is used for the corrected (output) signal generated by the subtraction unit or for the output signal of the subtraction unit.

In another embodiment the monitoring device further comprises an amplifier coupled between said light sensor and said filter unit for amplifying said sensor signal before filtering it.

In another embodiment the in-phase filter is configured to have a bandwidth corresponding to the signal bandwidth of the sensor signal. This provides that the signal-to-noise can be improved.

In an embodiment the in-phase filter and the out-of-phase filter are (nominally) identical filters. Because the main (in-phase) and out-of-phase signals pass through two instances of the same filter, the contribution of the (undesired) out-of-phase signal is the same in both branches so that through subtraction of the two different signals at the output of the two filters the desired output signal can be obtained.

Further embodiments of the proposed monitoring device, which are preferably implemented as a wrist-worn device for optical heart rate monitoring, although other implementations are possible, are directed to significantly reducing the impact of the sunlight.

In one embodiment said control unit is configured to control the intensity of light emitted by said light source to be in an predetermined intensity range and/ to control the gain of said analog-to-digital converter (in particular the gain of an amplifier included in said analog-to-digital converter) such that the signal level of an output signal of said monitoring device is within a predetermined range. This ensures that the signal level of the output signal that is used for heart rate detection is neither too high nor too low.

In another embodiment said control unit is configured to control the intensity of light emitted by said light source and/or the gain of said analog-to-digital converter such that the ratio between the difference between in-phase filter signal and out-of-phase filter signal to the out-of-phase filter signal is within a predetermined range. This ensures that the amount of light of the light source is not too small compared to ambient light (in particular sunlight).

In an alternative embodiment to achieve that the amount of light of the light source is not too small compared to ambient light (in particular sunlight) said control unit is configured to control the intensity of light emitted by said light source based on the out-of-phase filter signal such that the ratio between the difference between in-phase filter signal and out-of-phase filter signal to the out-of-phase filter signal is within a predetermined range.

BRIEF DESCRIPTION OF THE DRAWINGS

These and other aspects of the invention will be apparent from and elucidated with reference to the embodiment(s) described hereinafter. In the following drawings

Fig. 1 shows a schematic block diagram of an embodiment of a proposed monitoring device,
Fig. 2 shows a diagram illustrating aspects of the proposed monitoring method,
Fig. 3 shows a diagram of the spectrum of the sensor signal,
Fig. 4 shows a diagram of the transfer function of the proposed monitoring device,
Fig. 5 shows a schematic block diagram illustrating the signal processing with noise sources in the proposed monitoring device,
Fig. 6 shows an equivalent circuit diagram of an embodiment of a photo diode coupled to an amplifier,
Fig. 7 shows a diagram of the spectrum of the sensor signal including noise and of the output signal of the proposed monitoring device,
Fig. 8 shows a schematic block diagram of another embodiment of a proposed monitoring device, and
Fig. 9 shows a diagram of the light intensity of the light source in the embodiment shown in Fig. 8.

DETAILED DESCRIPTION OF THE INVENTION

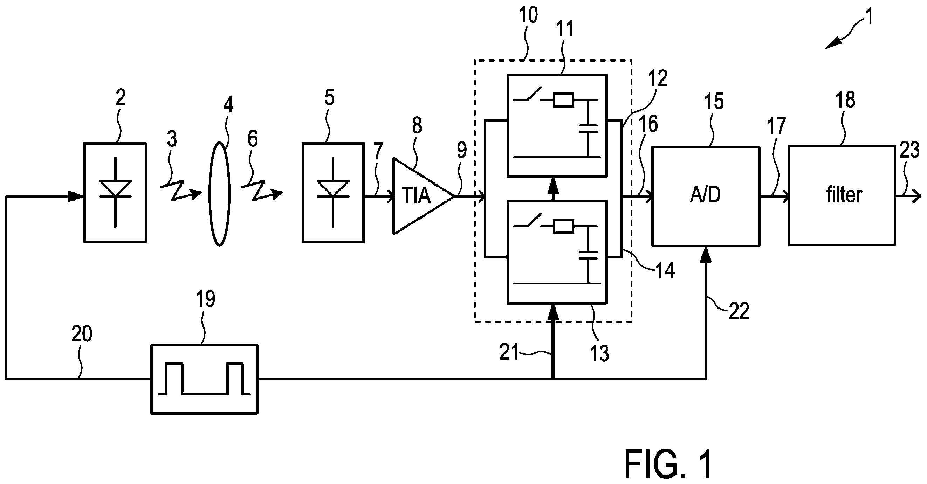

Fig. 1 shows a schematic block diagram of an embodiment of a monitoring device 1 according to the present invention. The monitoring device 1 comprises a light source 2 (e.g. an LED; in generally, the light source is meant hereinafter even if the term LED is used) for emitting light pulses 3 into tissue 4 of a living being, in particular a person (e.g. a patient, person doing sports, etc.) or an animal (e.g. in a zoo). The light source 2 may, e.g., be mounted on a finger clip or a watch worn by the living being. The device 1 further comprises a light sensor 5 (e.g. a photo diode) for receiving light 6 from said tissue 4 and generating a sensor signal 7 (e.g. a photo diode current). Optionally, an amplifier 8, in particular a transimpedance amplifier (TIA), is provided for amplifying the sensor signal 7 into an amplified sensor signal 9 (e.g. for amplifying the photo diode current into a voltage signal).

The (optionally amplified) sensor signal 9 is provided to a filter unit 10 for filtering said sensor signal 9. The filter unit 10 comprises a switched in-phase low-pass filter 11 (also called in-phase integrator in the following) for generating an in-phase filter signal 12 and a switched out-of-phase low-pass filter 13 (also called pre and post filter or pre and post integrator in the following) for generating an out-of-phase filter signal 14.

Optionally, an analog-to-digital (AD) converter 15 is provided for analog-to-digital converting the filter output signal 16 (comprising the in-phase filter signal 12 and the out-of-phase filter signal 14). The (optionally AD converted) filter output signal 17 is then forwarded to a subtraction unit 18 for subtracting the (optionally AD converted) out-of-phase filter signal 14 from the (optionally AD converted) in-phase filter signal 12 to obtain an output signal 23 that is used for heart rate detection.

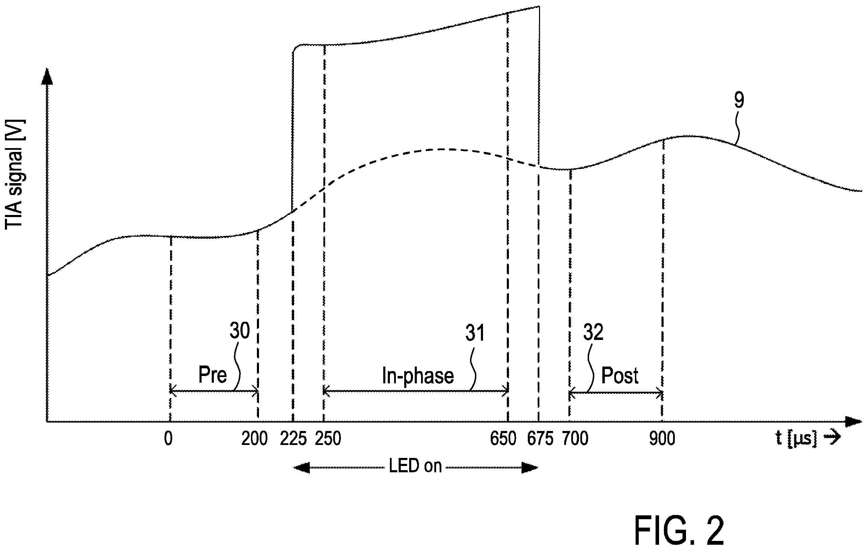

Finally, a control unit 19 is provided for controlling said light source 2 and said filter unit 10 (and, optionally, the AD converter 15) by use of control signals 20, 21, 22 such that the in-phase filter 11 is only switched on during a second time period (also called in-phase integration interval) while the light source 2 is switched on and that the out-of-phase filter 13 is switched on during a first and third time period (also called pre- and post-phase integration intervals) while the light source 2 is switched off. This is illustrated in Fig. 2.

Fig. 2 shows a diagram illustrating the timing of the pre-, in-phase and post-integration intervals 30, 31, 32 in relation to the LED on signal, i.e. the control signal 20. In particular, the amplified sensor signal 9 (also called TIA signal) in Volts is shown over time t in $\mu$s. are shown. The pre- and post integration intervals 30, 32 (also called first and third time periods) aim to create an interpolated version of the (optionally amplified) sensor signal 9 without the LED 2 on (i.e. aim to reconstruct the disturbance signal while the LED 2 is on). During the in-phase interval 31 (also called second time period) both the LED induced signal as well as the disturbance signal are integrated in the (optionally amplified) sensor signal 9.

Thus, generally, the light source 2 is pulsed by the control unit 19 (generally comprising a timing generator) at a modulation frequency $f_m$. The sensor 5 and TIA 8 receive and amplify the pulsed sensor signal 7 and offer it to the two switched filters 11, 13.

The in-phase switched filter 11 is switched while the light source 2 is on. The pre and post filter 13 (i.e. the out-of-phase filter) is switched on shortly before and shortly after the light source 2 is on. In other words, the time distance between the first time period 30 and the third time period 32 is short compared to the time period of the fastest disturbance signal to be suppressed, i.e. $t_{3rdperiod} - t_{1st\ period} << 1/f_{disturbance\_max}$. The total integration time for each filter 11, 13 is preferably the same. When not switched on, the filters 11, 13 retain their state.

The main parameters of the device 1 are:

- $f_m$: The LED modulation frequency;
- $t_{ON}$: The LED on time;
- d: the duty cycle, which follows from $f_m$ and $t_{ON}$: $d = t_{ON} \times f_m$; it determines the average LED power;
- R and C values of the switched integrators (filters) in the device.

The main performance parameters of the device 1 are:

- disturbance suppression: suppression of optical and EMI disturbances;
- signal to noise ratio;
- bandwidth;
- power consumption.

Ambient light and electromagnetic interference may disturb the sensor (photo diode) signal 7. Much of this disturbance occurs at DC, and at multiples of the net frequency (like 50/60 Hz, 100/120 Hz). To suppress this disturbance synchronous detection is applied.

Temporally, the pre and post filters 13 can be seen as estimators of the disturbance signal at the time the LED 2 is on. The estimator is the average of the disturbance signal during the pre- and the post-periods 30, 32. For disturbances that are sufficiently low frequent, the estimate will be accurate.

Spectrally, a synchronous detector can be viewed as a high pass filter, which blocks the disturbance signal below a frequency $F_{SD}$. The disturbance signal is offered to both the pre and post filter 13 and the in-phase filter 11, and the output is calculated as $y(n) = x(n-1)-\frac{1}{2}(x(n-2)+x(n))$. The sampling rate of this filter is e.g. $\approx$ 2500 Hz.

A signal processing model containing a disturbance signal a(t) may be used. For suppressing the disturbance signal, the pre and post integrator 13 should well estimate the disturbance signal at the time the LED 2 is on. For this, it is important that the integrators 11, 13 behave equal. A combination of two methods may be used:

The component values (R of a resistor and C of a capacitor) should match well between the two switched integrators 11, 13.

Calibration is applied to match the behaviour of the two switched integrators 11, 13.

It is preferred in an embodiment that the pre and post integration intervals 30, 32 are sufficiently close to the in-phase interval 31 (the local sampling frequency must be high enough). Otherwise higher frequencies in the disturbance signal are not well suppressed. Disturbance suppression is independent of whether or not resetting is applied in the switched integrators. This is so because in both cases, the signal processing for the disturbance signal is the same for both signal paths.

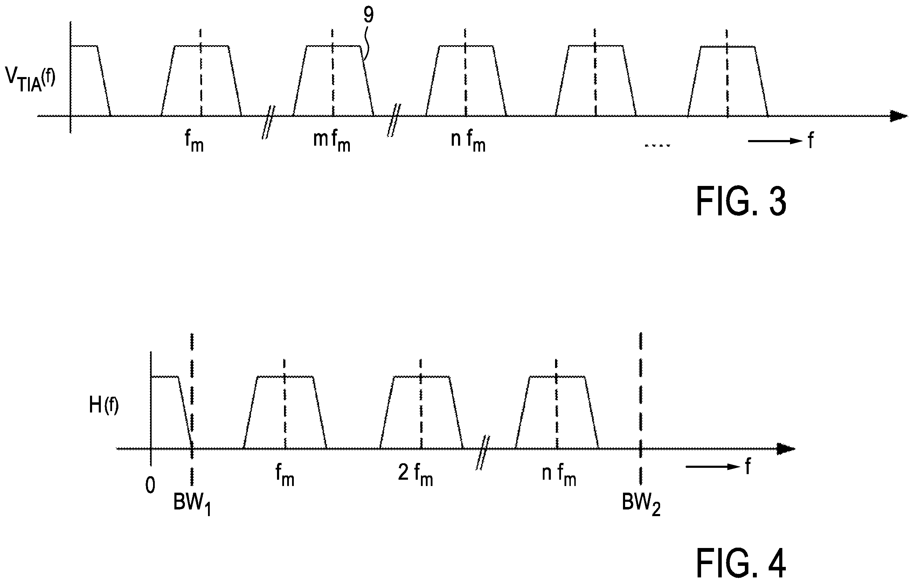

The baseband spectrum of the signal 3 through the tissue is modulated by the LED 2 with a frequency $f_m$. Due to this, the spectrum of the output signal 9 of the TIA 8 has the baseband spectrum repeated at multiples of $f_m$. The decay towards higher frequencies is determined by the LED duty cycle (according to a Fourier series). This is depicted in the signal diagram of Fig. 3 showing the spectrum of the output signal 9 of the TIA 8 indicated by $V_{TIA}$ over the frequency f.

Only when the integrators 11, 13 are switched, they integrate the input signal 9. When they are not switched they retain their state. The integrators 11, 13 function as a low pass filter. The switching duty cycle d affects the bandwidth of the filter. With state-space averaging, the bandwidth of the filter can be calculated:

$$BW_1 = \frac{d}{2\pi RC} \ [Hz] \qquad (1)$$

For higher frequencies, equation (1) does not hold. As soon as the period of the input signal 9 comes in the range of the switch on time ($t_{ON}$), then the natural RC time constant determines the behaviour and the signal is suppressed resulting in:

$$BW_2 \cong \frac{1}{2t_{ON}} \ [Hz] \qquad (2)$$

The transfer function H(f) of the device 1 over the frequency f is shown in Fig. 4. The transfer is limited by the bandwidth $BW_2$ and an additional low pass function $BW_1$ is present. This low pass filter is valid for all spectral repetitions at multiples of $f_m$. In other words, the bandwidth $BW_1$ is due to the switched low pass filter function and the bandwidth $BW_2$ is due to the duration of the switch on time.

Preferably, the pre period 30 (Tpre) and the post period 32 (Tpost) are identical in length to perform a symmetrical ambient integration:

$$\text{Tpre} = \text{Tpost} = 0.5 \times \text{Tin-phase.} \qquad (3)$$

The circuit then performs the following function:

$$M = S + A,$$
$$A' = 0.5 \times \text{Apre} + 0.5 \times \text{Apost} \qquad (4)$$

(this is the key of symmetrical ambient integration)

$$S' = M - A'.$$

The objective is to let A' be a very good estimate of A, such that S' = S, with M = signal from photo diode when LED is on,

S = signal of interest,
A = disturbance signal (ambient and noise),
A' = estimated A signal (weighed sum),
S' = estimated S.

Thereby, the weighing in equation (4) is due to equation (3).

In the following the noise behaviour shall be dealt with. A modified signal processing model is used to elaborate upon noise. Generally, the heart rate (heart pulse) part of the signal is of interest, which is roughly 1% of the total signal. To get a signal to noise ratio of say 40 dB on the signal, the overall signal to noise ratio must therefore be $\approx$ 40 dB + 20 x log(1/(1%)) = 40 dB + 40 dB = 80 dB.

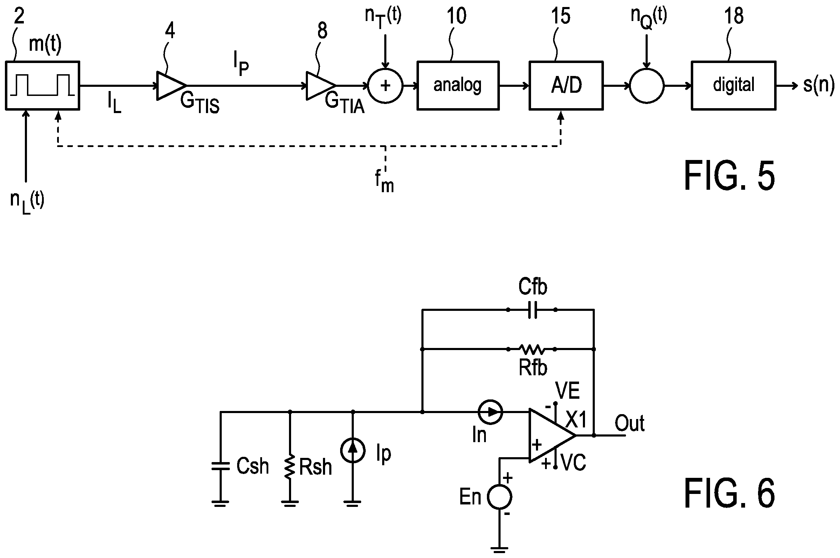

Fig. 5 shows a schematic block diagram illustrating the signal processing with noise sources in the proposed monitoring device. The following noise sources can be identified:

- $n_L(t)$: Noise on the LED light intensity. It is modulated by m(t), with $f_m$: $I_L = n_L(t)$ x m(t).
- $n_T(t)$: Noise that is referred to the TIA output. Consisting of TIA and photo diode noise.
- $n_Q(t)$: Noise from quantization by the A/D.

The LED noise $n_L(t)$ is modulated, so over the channel it occurs in multiple frequency bands. After detection these frequency bands are folded back to the baseband again. Therefore the noise in the frequency range 0 ... $BW_2$ is of importance. The signal to noise ratio of the LED signal is maintained across the channel. Both signal and noise are attenuated in the same way. A preferred requirement is to have > 80 dB for in-band noise.

For the photo diode the current $Ip$ is given comprising:

- Johnson noise from the shunt resistance of the photo diode $I_j = \sqrt{\frac{4kTB}{R_{sh}}}$.

- Shot noise caused by the dark current of the photo diode, which depends on the bias voltage $I_{sd} = \sqrt{2qI_dB}$.

- Light shot noise, which depends on the incident light on the photo diode $I_{sl} = \sqrt{2qI_lB}$.

For the TIA it is given:

- Voltage $E_n$ (nV/√Hz) and current noise In (fA/√Hz).

The total, output referred noise equates to:

$$E^2_{noise\_tot} = R^2_{fb}\left(I^2_j + I^2_{sd} + I^2_{sl} + I^2_n\right) + E^2_n(1 + \frac{Rfb}{Rsh})^2.$$

A corresponding equivalent circuit diagram of an embodiment of a photo diode coupled to an amplifier is depicted in Fig. 6

The noise from the TIA 8 and photo diode 5 are present independent of m(t) (except for the light quantisation noise). Because of this, the low frequent components are removed by the synchronous detection. Therefore only the noise in the frequency range approx. 500 Hz ... $BW_2$ is of importance. This is desired, as the 1/f noise is not contributing in this way. If a device is chosen without reset then an additional part of the noise is filtered away as shown in Fig. 4. To fully utilize the A/D converter 15, this noise floor should not exceed the A/D noise floor.

The A/D noise is white noise in the digital signal and independent of the chosen bandwidth. This noise can be reduced by oversampling. It would be relatively straightforward to do, as the signal is already captured by the sample and hold and there is sufficient time (provided the switches do not leak too much). This would allow the use of a lower resolution A/D converter.

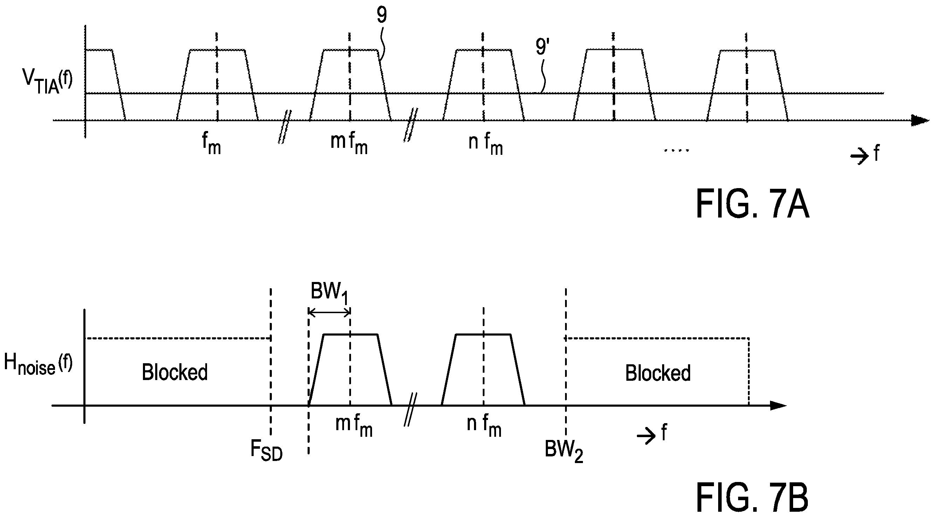

Assuming the noise from the TIA and photo diode are dominant, the signal to noise ratio (S/N ratio) shall be looked at. Fig. 7A shows the spectrum of the TIA signal 9 and Fig. 7B shows the output signal of the device 1. The TIA signal 9 shown in Fig. 7A consists of the signal spectrum repeated at $f_m$ and of noise. The signal transfer spectrum shown in Fig. 7B consists of bands with width $BW_1$, repeated at $f_m$, up to $BW_2$. The part above $BW_2$ is filtered out due to the low pass character of the pseudo integrator. The noise transfer spectrum is the same as the signal transfer spectrum, except that the lower part, up to $F_{SD}$ is filtered out by the synchronous detection ($F_{SD} \approx 500$ Hz). This results in:

$$\frac{S}{N} = 20 \cdot \log\frac{1}{\text{RMS}_{noise}} = 20 \cdot \log\frac{1}{\sqrt{BW}} + \text{constant}$$

The constant depends on the noise level in the channel and on the signal level (which are both independent of the detector parameters). It is assumed that the signal is fully contained within its band $BW_1$. The noise bandwidth BW is equal to the number of bands in ($BW_2$-$F_{SD}$) times the bandwidth of each band.

From this it can be derived that the signal to noise ratio can be improved by embodiments in which:

- $BW_1$ is chosen as small as possible to accommodate the signal bandwidth;
- $BW_2$ - $F_{SD}$ is small compared to $BW_2$.

In principle, the noise bandwidth can be reduced to 0 when $BW_2$ approaches $F_{SD}$. However, not all parameters are independent from each other as will be explained below.

After selecting $f_m$, $t_{ON}$, R and C were tuned to get BW1 equal to 8 Hz. Tradeoffs between LED power and S/N are possible in this way (increasing the LED power by increasing the intensity is a more efficient way of improving the S/N ratio). Intensity contributes linearly, while duration contributes by the square root. The relative S/N ratio was both calculated and simulated. The figures match, except for the bottom right. The calculation shows a +12 dB S/N improvement, while the simulated S/N improvement showed +6 dB.

The solutions differ on the following points:

- S/N (higher RC values in combination with long $t_{ON}$ give the best result).
- LED power (fm = 25 Hz requires the lowest).
- $F_{SD}$ ($t_{ON}$ = 250 $\mu$s has the highest, hence the best disturbance signal suppression).
- Aliasing ($f_m$ = 200 Hz is least susceptible to aliasing of the signal components).

The proposed monitoring device and method may advantageously be implemented as a wrist-worn optical heart rate monitor. Such a wrist-worn optical heart rate monitor comprises a light source (e.g. a LED) and a photo detector (light sensor). The light from the light source is led through the tissue of the living being (e.g. a person doing sports) to the photo detector, such that the heart rate may be determined. In wrist worn optical heart rate monitors for outdoor use, the influence of sunlight may play a major role. The sun is a very powerful light source and light from the sun may couple into the photo detector by leakage or by transmission through the tissue. In addition, due to rapid motion between sunlight and shadow, the amount of sunlight coupled into the photo detector may vary rapidly. This effect is worsened if the photo detector is sensitive for part of the sunlight spectrum, e.g. for green light. Filtering out can only be used for the non-green parts of the sunlight spectrum.

A problem with the sunlight coupling into the detector is that the heart rate signal may be severely disturbed. The heart rate is only a very minor part of the signal (like 0.2% to 2% of the total signal). Changes in the amount of sunlight that is coupled into the photo detector may be larger than that. Another problem with the sunlight may be that the control loop for the LED light intensity may become disturbed due to the sunlight. A high amount of sunlight coupling into the detector may lead to erroneous reduction of the LED light intensity. Hence, it is proposed in preferred embodiments to significantly reduce the impact of the sunlight.

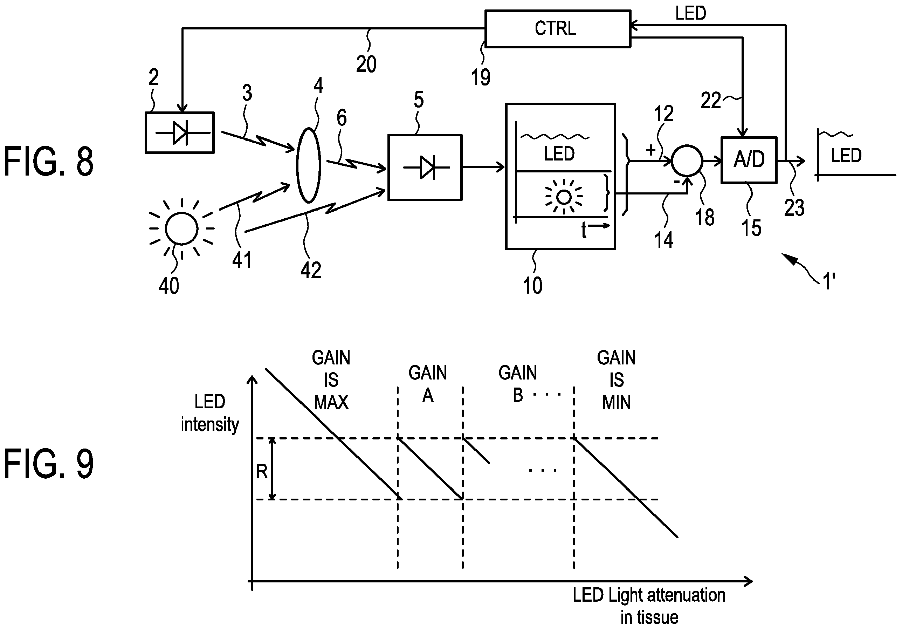

An embodiment of a monitoring device 1' according to the present invention comprising means for reducing the impact of the sunlight and being useful in an implementation as a wrist-worn optical heart rate monitor for outdoor use is depicted in Fig. 8. The photo detector 5 receives light 6 from the LED 2 and light 42 from the sun 40. The light 42 of the sun 40 is measured separately by averaging measurements prior and after the LED pulse, i.e. by averaging the measurements during the first and third time periods 30, 32, in the filter 10. The averaged sunlight value 14 is subtracted in the subtraction unit 18 from the photo detector signal 12, such that the LED part 25 of the signal remains. This remaining signal 25 is A/D converted and the resulting output signal 23 is used for heart rate (HR) detection.

This embodiment of the monitoring device 1' works fine for removing sunlight that is not varying very rapidly. But when the amount of sunlight ending up on the photo detector 5 varies very rapidly, then the ambient suppression is not infinitely good.. A small part of the sunlight variation ends up in the signal used for HR detection. This sets a lower limit to the needed LED intensity.

A controller 19 uses the output signal 23 from the A/D converter 15. A first objective of the controller 19 is to have a sufficient, but not a too high signal level going into the HR detection. It can do this by adjusting the LED intensity via LED control signal 20 and/or by adjusting the gain of a programmable gain amplifier inside the A/D converter 15 via converter control signal 22. Thus, the controller 19 has a certain freedom to use either of the two control signals 20, 22 to obtain a correct output signal level. For example, if the signal level is too low then it can either increase the gain or increase the LED intensity.

A second objective of the controller 19 is to let the amount of LED light ending up on the photo detector not be too small compared to the amount of sunlight. Otherwise, rapid sunlight variations may be too dominant compared to the LED light and the output signal may be disturbed. To achieve this second objective, the controller 19 has two possibilities. First, it may try to keep the LED intensity within a LED intensity range and regulate using the gain. Only if the gain is at one of the edges of the gain range then the LED is allowed to go outside its LED intensity range.

The behavior of the embodiment of the monitoring device 1' (using the above mentioned first possibility for achieving the second objective of the controller 19) is shown in the diagram depicted in Fig. 9. The LED intensity is drawn as a function of the LED light attenuation in the tissue. The monitoring device 1' attempts to keep the LED intensity within a range R (between the two horizontal dashed lines). Small changes in LED light attenuation are dealt with by adapting the LED intensity. In particular, the LED intensity is changed only when the signal goes outside a desired signal range, such that the number of LED intensity adaptations is minimized. This is done because an LED intensity adaptation leads to a disturbance in the signal. Larger variations that would cause the LED intensity to go out of the range are dealt with by changing the gain of the programmable gain amplifier in the AD converter 15. Only when the gain is at its maximum, the LED intensity is increased above the range. Likewise, when the gain is at its minimum, the LED intensity is decreased below the range.

In Fig. 9 the indices A and B shall indicate particular different gains. The idea of Fig. 9 is to show a sequence of m gains: $G_m$; $G_{m-1}$; $G_{m-2}$; ..., $G_2$; $G_1$, wherein $G_m$ is the maximum gain and $G_1$ is the minimum gain. In an embodiment it holds for the gains: $G_3 = 2$; $G_2 = 1$; $G_1 = 0.5$.

The present invention can e.g. be applied in a sports running watch or in consumer lifestyle products that e.g. use a band around the wrist and aim at detecting heart rate related physiological parameters. Further, it can be used in medical applications, both at home and in a hospital. In principle, the invention can be used for any system where the response to an own excitation must be measured and where additive disturbance signals are present. For instance, the invention can be applied in systems measuring the SpO2 rate (although such systems generally use two wavelengths, the principle of the present invention n is generally applicable).

The present invention is not limited to the use of a single light source and/or light of a single wavelength, but may also be applied in devices and methods using more than one light sources for emitting light pulses and/or light of more than a single wavelength, e.g. of a range of wavelengths or several separate wavelengths.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

**Claims**

**1.** Monitoring device comprising:

- a light source (2) for emitting light pulses (3) into tissue (4) of a living being,
- a light sensor (5) for receiving light (6) from said tissue and generating a sensor signal (7, 9),
- a filter unit (10) for filtering said sensor signal (7, 9), said filter unit comprising a switched in-phase low-pass filter (11) and a switched out-of-phase low-pass filter (13),
- a control unit (19) for controlling said light source (2) and said filter unit (10) such that the in-phase filter (11) is only switched on during a second time period (31) while the light source (2) is switched on and that the out-of-phase filter (13) is switched on during a first and third time period (30, 32) while the light source (2) is switched off, wherein said control unit (19) is configured to control said light source (2) and said filter unit (10) such that said first time period (30) is arranged shortly before the light source (2) gets switched on and said third time period (32) is arranged shortly after the light source (2) gets switched off,

**characterized in**:

- the out-of-phase filter (13) is configured to generate an ambient signal by integrating the sensor output (9) during the first and third time period (30, 32), and wherein the in-phase filter is configured to generate a main signal by integrating the sensor output during the second time period (31), and in further comprising:
- a subtraction unit (18) for generating a corrected signal by subtracting the ambient signal from the main signal.

**2.** Monitoring device as claimed in claim 1, wherein said control unit (19) is configured to control said filter unit (10) such that the second time period (31) has the same length as the sum of the first and third time periods (30, 32).

**3.** Monitoring device as claimed in claim 1, wherein said control unit (19) is configured to control said filter unit (10) such that the contribution of the disturbance signal during the second time period (31) is the same as the contribution of the disturbance signal during the first and third time periods (30, 32).

**4.** Monitoring device as claimed in claim 1, wherein said control unit (19) is configured to control said filter unit (10) such that the first time period (30) has the same length as the third time period (32).

**5.** Monitoring device as claimed in claim 1, wherein said control unit (19) is configured to control said light source (2) and said filter unit (10) such that said first time period (30) is arranged shortly before the light source (2) gets switched on and said third time period (32) is arranged shortly after the light source (2) gets switched off.

6. Monitoring device as claimed in claim 1,
   wherein said control unit (19) is configured to control said light source (2) and said filter unit (10) such that the time distance between the first time period and the third time period is short compared to the time period of the fastest disturbance signal to be suppressed.

7. Monitoring device as claimed in claim 1,
   wherein said control unit (19) is configured to control said light source (2) and said filter unit (10) such that the second time period is shorter than the time duration of a light pulse.

8. Monitoring device as claimed in claim 1,
   further comprising an analog-to-digital converter (15) coupled between said filter unit (10) and said subtraction unit (18) for analog-to-digital conversion of said in-phase filter signal (12) and said out-of-phase filter signal (14) before subtracting them or coupled to the output of said subtraction unit (18) for analog-to-digital conversion of the output signal of said subtraction unit (18).

9. Monitoring device as claimed in claim 1,
   further comprising an amplifier (8) coupled between said light sensor (5) and said filter unit (10) for amplifying said sensor signal (7) before filtering it.

10. Monitoring device as claimed in claim 1,
    wherein the in-phase filter (12) is configured to have a bandwidth corresponding to the signal bandwidth of the sensor signal (7).

11. Monitoring device as claimed in claim 1,
    wherein the in-phase filter (11) and the out-of-phase filter (13) are identical filters.

12. Monitoring device as claimed in claim 8,
    wherein said control unit (19) is configured to control the intensity of light emitted by said light source (2) to be in an predetermined intensity range (R) and to control the gain of said analog-to-digital converter (15) such that the signal level of an output signal (23) of said monitoring device is within a predetermined range.

13. Monitoring device as claimed in claim 1,
    wherein said control unit (19) is configured to control said light source (2) such that the light source (2) is alternately switched on during in-phase time periods (31) and off during out-of-phase time periods (30, 32) and to control said filter unit (10) such that the in-phase filter (11) is only switched on during the in-phase time periods (31) and that the out-of-phase filter (13) is switched on during the out-of-phase time periods (30, 32).

14. Monitoring device as claimed in claim 1,
    wherein said control unit (19) is configured to control said filter unit (10) and said light source (2) such that the sum of the in-phase time periods is nominally equal to the sum of the out-of-phase time periods.

15. Monitoring method comprising:

    - emitting light pulses (3) into tissue (4) of a living being,
    - receiving light (6) from said tissue (4) and generating a sensor signal (7),
    - filtering said sensor signal (7, 9) comprising switched in-phase low-pass filtering and switched out-of-phase low-pass filtering,
    - controlling said emitting of light pulses and said filtering of the sensor signal such that the in-phase filtering is only performed during a second time period (31) while a light pulse is emitted and that the out-of-phase filtering is performed during a first and third time period (30, 32) while no light pulse is emitted, wherein said emitting of light pulses and said filtering of the sensor signals is controlled such that said first time period (30) is arranged shortly before the light pulse is emitted and said third time period (32) is arranged shortly after the light pulse is emitted,

    **characterized in**:

- generating an ambient signal by integrating the out-of-phase filtered sensor signal during the first and third time period (30, 32),
- generating a main signal by integrating the in-phase filtered sensor signal during the second time period (31), and
- generating a corrected signal by subtracting the ambient signal from the main signal.

**Patentansprüche**

1. Überwachungsvorrichtung, umfassend:

   - eine Lichtquelle (2) zum Emittieren von Lichtpulsen (3) in Gewebe (4) eines Lebewesens,
   - einen Lichtsensor (5) zum Empfangen von Licht (6) aus dem genannten Gewebe und zum Erzeugen eines Sensorsignals (7, 9),
   - eine Filtereinheit (10) zum Filtern des genannten Sensorsignals (7, 9), wobei die genannte Filtereinheit einen geschalteten phasengleichen Tiefpassfilter (11) und einen geschalteten phasenverschobenen Tiefpassfilter (13) umfasst,
   - eine Steuereinheit (19) zum Steuern der genannten Lichtquelle (2) und der genannten Filtereinheit (10) derartig, dass der phasengleiche Filter (11) nur während einer zweiten Zeitperiode (31) eingeschaltet ist, während die Lichtquelle (2) eingeschaltet ist, und dass der phasenverschobene Filter (13) während einer ersten und dritten Zeitperiode (30, 32) eingeschaltet ist, während der die Lichtquelle (2) ausgeschaltet ist, wobei die genannte Steuereinheit (19) dafür konfiguriert ist, die genannte Lichtquelle (2) und die genannte Filtereinheit (10) derartig zu steuern, dass die genannte erste Zeitperiode (30) kurz vor dem Einschalten der Lichtquelle (2) vorgesehen ist und dass die genannte dritte Zeitperiode (32) kurz nach dem Ausschalten der Lichtquelle (2) vorgesehen ist,

   **dadurch gekennzeichnet, dass**

   - der phasenverschobene Filter (13) dafür konfiguriert ist, ein Umgebungssignal durch Integrieren der Sensorausgabe (9) während der ersten und dritten Zeitperiode (30, 32) zu erzeugen, und wobei der phasengleiche Filter dafür konfiguriert ist, ein Hauptsignal durch Integrieren der Sensorausgabe während der zweiten Zeitperiode (31) zu erzeugen, und dadurch, dass sie weiterhin umfasst:
   - eine Subtraktionseinheit (18) zum Erzeugen eines korrigierten Signals durch Subtrahieren des Umgebungssignals von dem Hauptsignal.

2. Überwachungsvorrichtung nach Anspruch 1, wobei die genannte Steuereinheit (19) dafür konfiguriert ist, die genannte Filtereinheit (10) derartig zu steuern, dass die zweite Zeitperiode (31) die gleiche Länge hat wie die Summe der ersten und der dritten Zeitperiode (30, 32).

3. Überwachungsvorrichtung nach Anspruch 1, wobei die genannte Steuereinheit (19) dafür konfiguriert ist, die genannte Filtereinheit (10) derartig zu steuern, dass der Beitrag des Störsignals während der zweiten Zeitperiode (31) der gleiche ist wie der Beitrag des Störsignals während der ersten und der dritten Zeitperiode (30, 32).

4. Überwachungsvorrichtung nach Anspruch 1, wobei die genannte Steuereinheit (19) dafür konfiguriert ist, die genannte Filtereinheit (10) derartig zu steuern, dass die erste Zeitperiode (30) die gleiche Länge hat wie die dritte Zeitperiode (32).

5. Überwachungsvorrichtung nach Anspruch 1, wobei die genannte Steuereinheit (19) dafür konfiguriert ist, die genannte Lichtquelle (2) und die genannte Filtereinheit (10) derartig zu steuern, dass die genannte erste Zeitperiode (30) kurz vor dem Einschalten der Lichtquelle (2) vorgesehen ist und die genannte dritte Zeitperiode (32) kurz nach dem Ausschalten der Lichtquelle (2) vorgesehen ist.

6. Überwachungsvorrichtung nach Anspruch 1, wobei die genannte Steuereinheit (19) dafür konfiguriert ist, die genannte Lichtquelle (2) und die genannte Filtereinheit (10) derartig zu steuern, dass der Zeitabstand zwischen der ersten Zeitperiode und der dritten Zeitperiode kurz im Vergleich zu der Zeitperiode des schnellsten zu unterdrückenden Störsignals ist.

7. Überwachungsvorrichtung nach Anspruch 1,

wobei die genannte Steuereinheit (19) dafür konfiguriert ist, die genannte Lichtquelle (2) und die genannte Filtereinheit (10) derartig zu steuern, dass die zweite Zeitperiode kürzer als die Zeitdauer eines Lichtpulses ist.

8. Überwachungsvorrichtung nach Anspruch 1,
weiterhin umfassend einen Analog-Digital-Umsetzer (15), der zwischen die genannte Filtereinheit (10) und die genannte Subtraktionseinheit (18) gekoppelt ist, um eine Analog-Digital-Umsetzung für das genannte phasengleiche Filtersignal (12) und das genannte phasenverschobene Filtersignal (14) durchzuführen, bevor sie subtrahiert werden, oder mit dem Ausgang der genannten Subtraktionseinheit (18) gekoppelt ist, um eine Analog-DigitalUmsetzung des Ausgangssignals der genannten Subtraktionseinheit (18) durchzuführen.

9. Überwachungsvorrichtung nach Anspruch 1,
weiterhin umfassend einen Verstärker (8), der zwischen den genannten Lichtsensor (5) und die genannte Filtereinheit (10) gekoppelt ist, um das genannte Sensorsignal (7) zu verstärken, bevor es gefiltert wird.

10. Überwachungsvorrichtung nach Anspruch 1,
wobei der phasengleiche Filter (12) dafür konfiguriert ist, eine Bandbreite zu haben, die der Signalbandbreite des Sensorsignals (7) entspricht.

11. Überwachungsvorrichtung nach Anspruch 1,
wobei der phasengleiche Filter (11) und der phasenverschobene Filter (13) identische Filter sind.

12. Überwachungsvorrichtung nach Anspruch 8,
wobei die genannte Steuereinheit (19) dafür konfiguriert ist, die Intensität des durch die Lichtquelle (2) emittierten Lichts derartig zu steuern, dass sie in einem vorgegebenen Intensitätsbereich (R) liegt, und die Verstärkung des genannten Analog-Digital-Umsetzers (15) derartig zu steuern, dass der Signalpegel eines Ausgangssignals (23) der genannten Überwachungsvorrichtung innerhalb eines vorgegebenen Bereichs liegt.

13. Überwachungsvorrichtung nach Anspruch 1,
wobei die genannte Steuereinheit (19) dafür konfiguriert ist, die genannte Lichtquelle (2) derartig zu steuern, dass die Lichtquelle (2) abwechselnd während phasengleicher Zeitperioden (31) eingeschaltet und während phasenverschobener Zeitperioden (30, 32) ausgeschaltet ist, und die genannte Filtereinheit (10) derartig zu steuern, dass der phasengleiche Filter (11) nur während der phasengleichen Zeitperioden (31) eingeschaltet ist und dass der phasenverschobene Filter (13) während der phasenverschobenen Zeitperioden (30, 32) eingeschaltet ist.

14. Überwachungsvorrichtung nach Anspruch 1,
wobei die genannte Steuereinheit (19) dafür konfiguriert ist, die genannte Filtereinheit (10) und die genannte Lichtquelle (2) derartig zu steuern, dass die Summe der phasengleichen Zeitperioden nominal gleich der Summe der phasenverschobenen Zeitperioden ist.

15. Überwachungsverfahren, umfassend:

- Emittieren von Lichtpulsen (3) in Gewebe (4) eines Lebewesens,
- Empfangen von Licht (6) aus dem genannten Gewebe (4) und Erzeugen eines Sensorsignals (7, 9),
- Filtern des genannten Sensorsignals (7, 9) umfassend geschaltete phasengleiche Tiefpassfilterung und geschaltete phasenverschobene Tiefpassfilterung,
- Steuern des genannten Emittierens von Lichtpulsen und des genannten Filtern des Sensorsignals derartig, dass die phasengleiche Filterung nur während einer zweiten Zeitperiode (31) durchgeführt wird, während ein Lichtpuls emittiert wird, und dass die phasenverschobene Filterung während einer ersten und dritten Zeitperiode (30, 32) durchgeführt wird, während der kein Lichtpuls emittiert wird, wobei das genannte Emittieren von Lichtpulsen und das genannte Filtern der Sensorsignale derartig gesteuert wird, dass die genannte erste Zeitperiode (30) kurz vor dem Emittieren des Lichtpulses vorgesehen ist und dass die genannte dritte Zeitperiode (32) kurz nach dem Emittieren der Lichtpulse vorgesehen ist,

**gekennzeichnet durch**

- das Erzeugen eines Umgebungssignals durch Integrieren des phasenverschobenen gefilterten Sensorsignals während der ersten und dritten Zeitperiode (30, 32),
- das Erzeugen eines Hauptsignals durch Integrieren des phasengleichen gefilterten Sensorsignals während der zweiten Zeitperiode (31), und
- das Erzeugen eines korrigierten Signals durch Subtrahieren des Umgebungssignals von dem Hauptsignal.

**Revendications**

1.  Dispositif de surveillance comprenant :

    - une source de lumière (2) pour émettre des impulsions de lumière (3) dans le tissu (4) d'un être vivant,
    - un capteur de lumière (5) pour recevoir de la lumière (6) à partir dudit tissu et générer un signal de capteur (7, 9),
    - une unité de filtre (10) pour filtrer ledit signal de capteur (7, 9), ladite unité de filtre comprenant un filtre passe-bas en phase commuté (11) et un filtre passe-bas hors phase commuté (13),
    - une unité de commande (19) pour commander ladite source de lumière (2) et ladite unité de filtre (10) de telle sorte que le filtre en phase (11) est uniquement activé pendant une deuxième période de temps (31) alors que la source de lumière (2) est activée et que le filtre hors phase (13) est activé pendant une première et une troisième période de temps (30, 32) alors que la source de lumière (2) est désactivée, dans lequel ladite unité de commande (19) est configurée pour commander ladite source de lumière (2) et ladite unité de filtre (10) de telle sorte que ladite première période de temps (30) est organisée juste avant que la source de lumière (2) soit activée et ladite troisième période de temps (32) est organisée juste après que la source de lumière (2) soit désactivée,

    **caractérisé en ce que**

    - le filtre hors phase (13) est configuré pour générer un signal ambiant en intégrant la sortie de capteur (9) pendant la première et la troisième période de temps (30, 32), et dans lequel le filtre en phase est configuré pour générer un signal principal en intégrant la sortie du capteur pendant la seconde période de temps (31), et comprenant en outre :
    - une unité de soustraction (18) pour générer un signal corrigé en soustrayant le signal ambiant du signal principal.

2.  Dispositif de surveillance selon la revendication 1,
    dans lequel ladite unité de commande (19) est configurée pour commander ladite unité de filtre (10) de telle sorte que la deuxième période de temps (31) ait la même longueur que la somme des première et troisième périodes de temps (30, 32).

3.  Dispositif de surveillance selon la revendication 1,
    dans lequel ladite unité de commande (19) est configurée pour commander ladite unité de filtre (10) de telle sorte que la contribution du signal de perturbation pendant la deuxième période de temps (31) est la même que la contribution du signal de perturbation pendant les première et troisième périodes de temps (30, 32).

4.  Dispositif de surveillance selon la revendication 1,
    dans lequel ladite unité de commande (19) est configurée pour commander ladite unité de filtre (10) de telle sorte que la première période de temps (30) a la même longueur que la troisième période de temps (32).

5.  Dispositif de surveillance selon la revendication 1,
    dans lequel ladite unité de commande (19) est configurée pour commander ladite source de lumière (2) et ladite unité de filtre (10) de telle sorte que ladite première période de temps (30) est organisée juste avant que la source de lumière (2) soit activée et que ladite troisième période de temps (32) est organisée juste après que la source de lumière (2) soit désactivée.

6.  Dispositif de surveillance selon la revendication 1,
    dans lequel ladite unité de commande (19) est configurée pour commander ladite source de lumière (2) et ladite unité de filtre (10) de telle sorte que la distance en temps entre la première période de temps et la troisième période de temps est courte par rapport à la période de temps du signal de perturbation le plus rapide devant être supprimé.

7.  Dispositif de surveillance selon la revendication 1,
    dans lequel ladite unité de commande (19) est configurée pour commander ladite source de lumière (2) et ladite unité de filtre (10) de telle sorte que la seconde période de temps est plus courte que la durée en temps d'une impulsion de lumière.

8. Dispositif de surveillance selon la revendication 1,
comprenant en outre un convertisseur analogique-numérique (15) couplé entre ladite unité de filtre (10) et ladite unité de soustraction (18) pour la conversion analogique-numérique dudit signal de filtre en phase (12) et dudit signal de filtre hors phase (14) avant de les soustraire ou couplé à la sortie de ladite unité de soustraction (18) pour la conversion analogique-numérique du signal de sortie de ladite unité de soustraction (18).

9. Dispositif de surveillance selon la revendication 1,
comprenant en outre un amplificateur (8) couplé entre ledit capteur de lumière (5) et ladite unité de filtre (10) pour amplifier ledit signal de capteur (7) avant de le filtrer.

10. Dispositif de surveillance selon la revendication 1,
dans lequel le filtre en phase (12) est configuré pour avoir une largeur de bande correspondant à la largeur de bande de signal du signal de capteur (7).

11. Dispositif de surveillance selon la revendication 1,
dans lequel le filtre en phase (11) et le filtre hors phase (13) sont des filtres identiques.

12. Dispositif de surveillance selon la revendication 8,
dans lequel ladite unité de commande (19) est configurée pour commander l'intensité de la lumière émise par ladite source de lumière (2) pour qu'elle soit dans une plage d'intensité prédéterminée (R) et pour commander le gain dudit convertisseur analogique-numérique (15) de sorte que le niveau de signal d'un signal de sortie (23) dudit dispositif de surveillance se trouve dans une plage prédéterminée.

13. Dispositif de surveillance selon la revendication 1,
dans lequel ladite unité de commande (19) est configurée pour commander ladite source de lumière (2) de telle sorte que la source de lumière (2) est activée en alternance pendant des périodes de temps en phase (31) et désactivée pendant des périodes de temps hors phase (30, 32) et pour commander ladite unité de filtre (10) de telle sorte que le filtre en phase (11) est uniquement activé pendant les périodes de temps en phase (31) et que le filtre hors phase (13) est activé pendant les périodes de temps hors phase (30, 32).

14. Dispositif de surveillance selon la revendication 1,
dans lequel ladite unité de commande (19) est configurée pour commander ladite unité de filtre (10) et ladite source de lumière (2) de telle sorte que la somme des périodes de temps en phase est nominalement égale à la somme des périodes de temps hors phase.

15. Procédé de surveillance comprenant :

- l'émission d'impulsions de lumière (3) dans le tissu (4) d'un être vivant,
- la réception de lumière (6) à partir dudit tissu (4) et la génération d'un signal de capteur (7,9),
- le filtrage dudit signal de capteur (7, 9) comprenant le filtrage passe-bas en phase commuté et le filtrage passe-bas hors phase commuté,
- la commande de ladite émission d'impulsions de lumière et dudit filtrage du signal de capteur de telle sorte que le filtrage en phase est uniquement effectué pendant une deuxième période de temps (31) alors qu'une impulsion de lumière est émise et que le filtrage hors phase est effectué pendant une première et une troisième période de temps (30, 32) alors qu'aucune impulsion de lumière n'est émise, dans lequel ladite émission d'impulsions de lumière et ledit filtrage des signaux de capteur est commandé de telle sorte que ladite première période de temps (30) est organisée juste avant que l'impulsion de lumière soit émise et que ladite troisième période de temps (32) est organisée juste après que l'impulsion de lumière soit émise,

**caractérisé en ce que** :

- la génération d'un signal ambiant en intégrant le signal de capteur filtré hors phase pendant les première et troisième périodes de temps (30, 32),
- la génération d'un signal principal en intégrant le signal de capteur filtré en phase pendant la deuxième période de temps (31), et
- la génération d'un signal corrigé en soustrayant le signal ambiant du signal principal.

FIG. 1

FIG. 2

EP 2 861 131 B1

FIG. 3

FIG. 4

FIG. 5

FIG. 6

EP 2 861 131 B1

FIG. 7A

$V_{TIA}(f)$

$f_m$    $m f_m$    $n f_m$    $\rightarrow f$

9    9'

FIG. 7B

$H_{noise}(f)$

Blocked

$F_{SD}$    $BW_1$    $m f_m$    $n f_m$    $BW_2$    $\rightarrow f$

Blocked

FIG. 8

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5846190 A
- US 6912413 B